# EUROPEAN PATENT APPLICATION

(11) **EP 1 076 091 A1**
(43) Date of publication of application: **14.02.2001**
(21) Application number: 99870171.8
(22) Date of filing: 09.08.1999
(51) Int. Cl.: C12N 15/12, A61K 38/17, A61K 31/40, A61K 31/70, G01N 33/50, A61P 9/00, A61P 35/00

(54) **Medicament for the prevention and/or the treatment of ischemic heart and peripheral vascular diseases, tumour and wounds**

(71) Applicant: UNIVERSITE CATHOLIQUE DE LOUVAIN, B-1348 Louvain-la-Neuve (BE)
(72) Inventor: Feron, Olivier, 1150 Brussels (BE); Balligand, Jean-Luc, 1950 Kraainem (BE)
(74) Representative: Van Malderen, Joelle

(57) **Abstract**

The present invention is related to the use of a compound or a pharmaceutical composition for the prevention and/or the treatment of ischemic heart and peripheral vascular diseases including cerebral diseases, tumour development and wound healing, wherein said compound or composition being able to modulate positively or negatively in a patient:
- either the endogenous caveolin-1 binding to its active site(s),
- or the concentration of endogenous caveolin-1.

SEQUENCE LISTING

<110> UNIVERSITE CATHOLIQUE DE LOUVAIN

<120> USE OF COMPOUND OR PHARMACEUTICAL COMPOSITION FOR THE
      PREVENTION AND/OR THE TREATMENT OF ISCHEMIC HEART AND
      PERIPHERAL VASCULAR DISEASES, TUMOUR DEVELOPMENT AND
      FOR WOUND HEALING

<130> P.UCL.72/EP

<140>
<141>

<160> 86

<170> PatentIn Ver. 2.1

<210> 1
<211> 537
<212> DNA
<213> Homo sapiens

<400> 1
atgtctgggg gcaaatacgt agactcggag ggacatctct acaccgttcc catccgggaa 60
cagggcaaca tctacaagcc caacaacaag gccatggcag acgagctgag cgagaagcaa 120
gtgtacgacg cgcacaccaa ggagatcgac ctggtcaacc gcgaccctaa acacctcaac 180
gatgacgtgg tcaagattga ctttgaagat gtgattgcag aaccagaagg gacacacagt 240
tttcacggca tttggaaggc cagcttcacc accttcactg tgacgaaata ctggttttac 300
cgcttgctgt ctgccctctt tggcatcccg atggcactca tctggggcat ttacttcgcc 360
attctctctt tcctgcacat ctgggcagtt gtaccatgca ttaagagctt cctgattgag 420
attcagtgca ccagccgtgt ctattccatc tacgtccaca ccgtctgtga cccactcttt 480
gaagctgttg ggaaaatatt cagcaatgtc cgcatcaact tgcagaaaga aatataa    537


<210> 2
<211> 20
<212> PRT
<213> Homo sapiens

<400> 2
His Gly Ile Trp Lys Ala Ser Phe Thr Thr Phe Thr Val Thr Lys Tyr
  1               5                  10                  15

Trp Phe Tyr Arg
             20

<210> 3
<211> 22
<212> PRT
<213> Homo sapiens

<400> 3
Lys Ser Phe Leu Ile Glu Ile Gln Cys Thr Ser Arg Val Tyr Ser Ile
1               5                   10                  15

Tyr Val His Thr Val Cys
            20

<210> 4
<211> 10
<212> PRT
<213> Artificial Sequence

<220>
<223> Description of Artificial Sequence: caveolin
      binding motif

<400> 4
Phe Pro Ala Ala Pro Phe Ser Gly Trp Tyr
1               5                   10

<210> 5
<211> 40
<212> DNA
<213> Artificial Sequence

<220>
<223> Description of Artificial Sequence: partial
      antisense  sequence of human caveolin-1

<400> 5
gagtctacgt atttgccccc agacatgctg gcccgtggct                            40

<210> 6
<211> 10
<212> PRT
<213> Artificial Sequence

<220>
<223> Description of Artificial Sequence: caveolin
      binding motif

<400> 6
Phe Xaa Phe Xaa Xaa Xaa Xaa Phe Xaa Phe
  1               5                   10

<210> 7
<211> 10
<212> PRT
<213> Artificial Sequence

<220>
<223> Description of Artificial Sequence: caveolin
      binding motif

<400> 7
Phe Xaa Phe Xaa Xaa Xaa Xaa Phe Xaa Tyr
  1               5                   10

<210> 8
<211> 10
<212> PRT
<213> Artificial Sequence

<220>
<223> Description of Artificial Sequence: caveolin
      binding motif

<400> 8
Phe Xaa Phe Xaa Xaa Xaa Xaa Tyr Xaa Tyr
  1               5                   10

<210> 9
<211> 10
<212> PRT
<213> Artificial Sequence

<220>
<223> Description of Artificial Sequence: caveolin
      binding motif

<400> 9
Phe Xaa Tyr Xaa Xaa Xaa Xaa Tyr Xaa Tyr
1               5                   10

<210> 10
<211> 10
<212> PRT
<213> Artificial Sequence

<220>
<223> Description of Artificial Sequence: caveolin
      binding motif

<400> 10
Phe Xaa Trp Xaa Xaa Xaa Xaa Tyr Xaa Tyr
1               5                   10

<210> 11
<211> 10
<212> PRT
<213> Artificial Sequence

<220>
<223> Description of Artificial Sequence: caveolin
      binding motif

<400> 11
Phe Xaa Phe Xaa Xaa Xaa Xaa Trp Xaa Tyr
1               5                   10

<210> 12
<211> 10
<212> PRT
<213> Artificial Sequence

<220>
<223> Description of Artificial Sequence: caveolin
      binding motif

<400> 12
Phe Xaa Tyr Xaa Xaa Xaa Xaa Trp Xaa Tyr

1                   5                   10

<210> 13
<211> 10
<212> PRT
<213> Artificial Sequence

<220>
<223> Description of Artificial Sequence: caveolin
      binding motif

<400> 13
Phe Xaa Trp Xaa Xaa Xaa Xaa Trp Xaa Tyr
  1               5                   10

<210> 14
<211> 10
<212> PRT
<213> Artificial Sequence

<220>
<223> Description of Artificial Sequence: caveolin
      binding motif

<400> 14
Phe Xaa Tyr Xaa Xaa Xaa Xaa Phe Xaa Tyr
  1               5                   10

<210> 15
<211> 10
<212> PRT
<213> Artificial Sequence

<220>
<223> Description of Artificial Sequence: caveolin
      binding motif

<400> 15
Phe Xaa Trp Xaa Xaa Xaa Xaa Phe Xaa Tyr
  1               5                   10

<210> 16
<211> 10
<212> PRT
<213> Artificial Sequence

<220>
<223> Description of Artificial Sequence: caveolin
      binding motif

<400> 16
Phe Xaa Phe Xaa Xaa Xaa Xaa Phe Xaa Trp
  1               5                   10

<210> 17
<211> 10
<212> PRT
<213> Artificial Sequence

<220>
<223> Description of Artificial Sequence: caveolin
      binding motif

<400> 17
Phe Xaa Phe Xaa Xaa Xaa Xaa Tyr Xaa Trp
  1               5                   10

<210> 18
<211> 10
<212> PRT
<213> Artificial Sequence

<220>
<223> Description of Artificial Sequence: caveolin
      binding motif

<400> 18
Phe Xaa Tyr Xaa Xaa Xaa Xaa Tyr Xaa Trp
  1               5                   10

<210> 19
<211> 10

<212> PRT
<213> Artificial Sequence

<220>
<223> Description of Artificial Sequence: caveolin
      binding motif

<400> 19
Phe Xaa Trp Xaa Xaa Xaa Xaa Tyr Xaa Trp
  1               5                   10

<210> 20
<211> 10
<212> PRT
<213> Artificial Sequence

<220>
<223> Description of Artificial Sequence: caveolin
      binding motif

<400> 20
Phe Xaa Phe Xaa Xaa Xaa Xaa Trp Xaa Trp
  1               5                   10

<210> 21
<211> 10
<212> PRT
<213> Artificial Sequence

<220>
<223> Description of Artificial Sequence: caveolin
      binding motif

<400> 21
Phe Xaa Tyr Xaa Xaa Xaa Xaa Trp Xaa Trp
  1               5                   10

<210> 22
<211> 10
<212> PRT
<213> Artificial Sequence

<220>
<223> Description of Artificial Sequence: caveolin
      binding motif

<400> 22
Phe Xaa Trp Xaa Xaa Xaa Xaa Trp Xaa Trp
  1               5                  10

<210> 23
<211> 10
<212> PRT
<213> Artificial Sequence

<220>
<223> Description of Artificial Sequence: caveolin
      binding motif

<400> 23
Phe Xaa Tyr Xaa Xaa Xaa Xaa Phe Xaa Trp
  1               5                  10

<210> 24
<211> 10
<212> PRT
<213> Artificial Sequence

<220>
<223> Description of Artificial Sequence: caveolin
      binding motif

<400> 24
Phe Xaa Trp Xaa Xaa Xaa Xaa Phe Xaa Trp
  1               5                  10

<210> 25
<211> 10
<212> PRT
<213> Artificial Sequence

<220>
<223> Description of Artificial Sequence: caveolin
      binding motif

<400> 25
Phe Xaa Phe Xaa Xaa Xaa Xaa Tyr Xaa Phe
1               5                   10

<210> 26
<211> 10
<212> PRT
<213> Artificial Sequence

<220>
<223> Description of Artificial Sequence: caveolin
      binding motif

<400> 26
Phe Xaa Tyr Xaa Xaa Xaa Xaa Tyr Xaa Phe
1               5                   10

<210> 27
<211> 10
<212> PRT
<213> Artificial Sequence

<220>
<223> Description of Artificial Sequence: caveolin
      binding motif

<400> 27
Phe Xaa Trp Xaa Xaa Xaa Xaa Tyr Xaa Phe
1               5                   10

<210> 28
<211> 10
<212> PRT
<213> Artificial Sequence

<220>
<223> Description of Artificial Sequence: caveolin
      binding motif

<400> 28
Phe Xaa Phe Xaa Xaa Xaa Xaa Trp Xaa Phe

1                   5                       10

<210> 29
<211> 10
<212> PRT
<213> Artificial Sequence

<220>
<223> Description of Artificial Sequence: caveolin
      binding motif

<400> 29
Phe Xaa Tyr Xaa Xaa Xaa Xaa Trp Xaa Phe
  1               5                   10

<210> 30
<211> 10
<212> PRT
<213> Artificial Sequence

<220>
<223> Description of Artificial Sequence: caveolin
      binding motif

<400> 30
Phe Xaa Trp Xaa Xaa Xaa Xaa Trp Xaa Phe
  1               5                   10

<210> 31
<211> 10
<212> PRT
<213> Artificial Sequence

<220>
<223> Description of Artificial Sequence: caveolin
      binding motif

<400> 31
Phe Xaa Tyr Xaa Xaa Xaa Xaa Phe Xaa Phe
  1               5                   10

<210> 32
<211> 10
<212> PRT
<213> Artificial Sequence

<220>
<223> Description of Artificial Sequence: caveolin
      binding motif

<400> 32
Phe Xaa Trp Xaa Xaa Xaa Xaa Phe Xaa Phe
  1               5                   10

<210> 33
<211> 10
<212> PRT
<213> Artificial Sequence

<220>
<223> Description of Artificial Sequence: caveolin
      binding motif

<400> 33
Tyr Xaa Phe Xaa Xaa Xaa Xaa Phe Xaa Phe
  1               5                   10

<210> 34
<211> 10
<212> PRT
<213> Artificial Sequence

<220>
<223> Description of Artificial Sequence: caveolin
      binding motif

<400> 34
Tyr Xaa Phe Xaa Xaa Xaa Xaa Phe Xaa Tyr
  1               5                   10

<210> 35
<211> 10

<212> PRT
<213> Artificial Sequence

<220>
<223> Description of Artificial Sequence: caveolin
      binding motif

<400> 35
Tyr Xaa Phe Xaa Xaa Xaa Xaa Tyr Xaa Tyr
  1               5                   10

<210> 36
<211> 10
<212> PRT
<213> Artificial Sequence

<220>
<223> Description of Artificial Sequence: caveolin
      binding motif

<400> 36
Tyr Xaa Tyr Xaa Xaa Xaa Xaa Tyr Xaa Tyr
  1               5                   10

<210> 37
<211> 10
<212> PRT
<213> Artificial Sequence

<220>
<223> Description of Artificial Sequence: caveolin
      binding motif

<400> 37
Tyr Xaa Trp Xaa Xaa Xaa Xaa Tyr Xaa Tyr
  1               5                   10

<210> 38
<211> 10
<212> PRT
<213> Artificial Sequence

<220>
<223> Description of Artificial Sequence: caveolin
      binding motif

<400> 38
Tyr Xaa Phe Xaa Xaa Xaa Xaa Trp Xaa Tyr
  1               5                  10

<210> 39
<211> 10
<212> PRT
<213> Artificial Sequence

<220>
<223> Description of Artificial Sequence: caveolin
      binding motif

<400> 39
Tyr Xaa Tyr Xaa Xaa Xaa Xaa Trp Xaa Tyr
  1               5                  10

<210> 40
<211> 10
<212> PRT
<213> Artificial Sequence

<220>
<223> Description of Artificial Sequence: caveolin
      binding motif

<400> 40
Tyr Xaa Trp Xaa Xaa Xaa Xaa Trp Xaa Tyr
  1               5                  10

<210> 41
<211> 10
<212> PRT
<213> Artificial Sequence

<220>
<223> Description of Artificial Sequence: caveolin
      binding motif

<400> 41
Tyr Xaa Phe Xaa Xaa Xaa Xaa Phe Xaa Trp
1               5                   10

<210> 42
<211> 10
<212> PRT
<213> Artificial Sequence

<220>
<223> Description of Artificial Sequence: caveolin
      binding motif

<400> 42
Tyr Xaa Phe Xaa Xaa Xaa Xaa Tyr Xaa Trp
1               5                   10

<210> 43
<211> 10
<212> PRT
<213> Artificial Sequence

<220>
<223> Description of Artificial Sequence: caveolin
      binding motif

<400> 43
Tyr Xaa Tyr Xaa Xaa Xaa Xaa Tyr Xaa Trp
1               5                   10

<210> 44
<211> 10
<212> PRT
<213> Artificial Sequence

<220>
<223> Description of Artificial Sequence: caveolin
      binding motif

<400> 44
Tyr Xaa Trp Xaa Xaa Xaa Xaa Tyr Xaa Trp

1                   5                   10

<210> 45
<211> 10
<212> PRT
<213> Artificial Sequence

<220>
<223> Description of Artificial Sequence: caveolin
      binding motif

<400> 45
Tyr Xaa Phe Xaa Xaa Xaa Xaa Trp Xaa Trp
  1               5                   10

<210> 46
<211> 10
<212> PRT
<213> Artificial Sequence

<220>
<223> Description of Artificial Sequence: caveolin
      binding motif

<400> 46
Tyr Xaa Tyr Xaa Xaa Xaa Xaa Trp Xaa Trp
  1               5                   10

<210> 47
<211> 10
<212> PRT
<213> Artificial Sequence

<220>
<223> Description of Artificial Sequence: caveolin
      binding motif

<400> 47
Tyr Xaa Trp Xaa Xaa Xaa Xaa Trp Xaa Trp
  1               5                   10

<210> 48
<211> 10
<212> PRT
<213> Artificial Sequence

<220>
<223> Description of Artificial Sequence: caveolin
      binding motif

<400> 48
Tyr Xaa Phe Xaa Xaa Xaa Xaa Tyr Xaa Phe
  1               5                   10

<210> 49
<211> 10
<212> PRT
<213> Artificial Sequence

<220>
<223> Description of Artificial Sequence: caveolin
      binding motif

<400> 49
Tyr Xaa Tyr Xaa Xaa Xaa Xaa Tyr Xaa Phe
  1               5                   10

<210> 50
<211> 10
<212> PRT
<213> Artificial Sequence

<220>
<223> Description of Artificial Sequence: caveolin
      binding motif

<400> 50
Tyr Xaa Trp Xaa Xaa Xaa Xaa Tyr Xaa Phe
  1               5                   10

<210> 51
<211> 10

<212> PRT
<213> Artificial Sequence

<220>
<223> Description of Artificial Sequence: caveolin
      binding motif

<400> 51
Tyr Xaa Phe Xaa Xaa Xaa Xaa Trp Xaa Phe
  1               5                  10

<210> 52
<211> 10
<212> PRT
<213> Artificial Sequence

<220>
<223> Description of Artificial Sequence: caveolin
      binding motif

<400> 52
Tyr Xaa Tyr Xaa Xaa Xaa Xaa Trp Xaa Phe
  1               5                  10

<210> 53
<211> 10
<212> PRT
<213> Artificial Sequence

<220>
<223> Description of Artificial Sequence: caveolin
      binding motif

<400> 53
Tyr Xaa Trp Xaa Xaa Xaa Xaa Trp Xaa Phe
  1               5                  10

<210> 54
<211> 10
<212> PRT
<213> Artificial Sequence

<220>
<223> Description of Artificial Sequence: caveolin
      binding motif

<400> 54
Tyr Xaa Tyr Xaa Xaa Xaa Xaa Phe Xaa Phe
  1               5                   10

<210> 55
<211> 10
<212> PRT
<213> Artificial Sequence

<220>
<223> Description of Artificial Sequence: caveolin
      binding motif

<400> 55
Tyr Xaa Trp Xaa Xaa Xaa Xaa Phe Xaa Phe
  1               5                   10

<210> 56
<211> 10
<212> PRT
<213> Artificial Sequence

<220>
<223> Description of Artificial Sequence: caveolin
      binding motif

<400> 56
Tyr Xaa Tyr Xaa Xaa Xaa Xaa Phe Xaa Tyr
  1               5                   10

<210> 57
<211> 10
<212> PRT
<213> Artificial Sequence

<220>
<223> Description of Artificial Sequence: caveolin
      binding motif

<400> 57
Tyr Xaa Trp Xaa Xaa Xaa Xaa Phe Xaa Tyr
1               5                   10

<210> 58
<211> 10
<212> PRT
<213> Artificial Sequence

<220>
<223> Description of Artificial Sequence: caveolin
      binding motif

<400> 58
Tyr Xaa Tyr Xaa Xaa Xaa Xaa Phe Xaa Trp
1               5                   10

<210> 59
<211> 10
<212> PRT
<213> Artificial Sequence

<220>
<223> Description of Artificial Sequence: caveolin
      binding motif

<400> 59
Tyr Xaa Trp Xaa Xaa Xaa Xaa Phe Xaa Trp
1               5                   10

<210> 60
<211> 10
<212> PRT
<213> Artificial Sequence

<220>
<223> Description of Artificial Sequence: caveolin
      binding motif

<400> 60
Trp Xaa Phe Xaa Xaa Xaa Xaa Phe Xaa Phe

1               5                   10

<210> 61
<211> 10
<212> PRT
<213> Artificial Sequence

<220>
<223> Description of Artificial Sequence: caveolin
      binding motif

<400> 61
Trp Xaa Phe Xaa Xaa Xaa Xaa Phe Xaa Tyr
  1               5                   10

<210> 62
<211> 10
<212> PRT
<213> Artificial Sequence

<220>
<223> Description of Artificial Sequence: caveolin
      binding motif

<400> 62
Trp Xaa Phe Xaa Xaa Xaa Xaa Tyr Xaa Tyr
  1               5                   10

<210> 63
<211> 10
<212> PRT
<213> Artificial Sequence

<220>
<223> Description of Artificial Sequence: caveolin
      binding motif

<400> 63
Trp Xaa Tyr Xaa Xaa Xaa Xaa Tyr Xaa Tyr
  1               5                   10

<210> 64
<211> 10
<212> PRT
<213> Artificial Sequence

<220>
<223> Description of Artificial Sequence: caveolin
      binding motif

<400> 64
Trp Xaa Trp Xaa Xaa Xaa Xaa Tyr Xaa Tyr
  1               5                   10

<210> 65
<211> 10
<212> PRT
<213> Artificial Sequence

<220>
<223> Description of Artificial Sequence: caveolin
      binding motif

<400> 65
Trp Xaa Phe Xaa Xaa Xaa Xaa Trp Xaa Tyr
  1               5                   10

<210> 66
<211> 10
<212> PRT
<213> Artificial Sequence

<220>
<223> Description of Artificial Sequence: caveolin
      binding motif

<400> 66
Trp Xaa Tyr Xaa Xaa Xaa Xaa Trp Xaa Tyr
  1               5                   10

<210> 67
<211> 10

<212> PRT
<213> Artificial Sequence

<220>
<223> Description of Artificial Sequence: caveolin
      binding motif

<400> 67
Trp Xaa Trp Xaa Xaa Xaa Xaa Trp Xaa Tyr
  1               5                   10

<210> 68
<211> 10
<212> PRT
<213> Artificial Sequence

<220>
<223> Description of Artificial Sequence: caveolin
      binding motif

<400> 68
Trp Xaa Phe Xaa Xaa Xaa Xaa Phe Xaa Trp
  1               5                   10

<210> 69
<211> 10
<212> PRT
<213> Artificial Sequence

<220>
<223> Description of Artificial Sequence: caveolin
      binding motif

<400> 69
Trp Xaa Phe Xaa Xaa Xaa Xaa Tyr Xaa Trp
  1               5                   10

<210> 70
<211> 10
<212> PRT
<213> Artificial Sequence

<220>
<223> Description of Artificial Sequence: caveolin
      binding motif

<400> 70
Trp Xaa Tyr Xaa Xaa Xaa Xaa Tyr Xaa Trp
  1               5                   10

<210> 71
<211> 10
<212> PRT
<213> Artificial Sequence

<220>
<223> Description of Artificial Sequence: caveolin
      binding motif

<400> 71
Trp Xaa Trp Xaa Xaa Xaa Xaa Tyr Xaa Trp
  1               5                   10

<210> 72
<211> 10
<212> PRT
<213> Artificial Sequence

<220>
<223> Description of Artificial Sequence: caveolin
      binding motif

<400> 72
Trp Xaa Phe Xaa Xaa Xaa Xaa Trp Xaa Trp
  1               5                   10

<210> 73
<211> 10
<212> PRT
<213> Artificial Sequence

<220>
<223> Description of Artificial Sequence: caveolin
      binding motif

<400> 73
Trp Xaa Tyr Xaa Xaa Xaa Xaa Trp Xaa Trp
1               5                   10

<210> 74
<211> 10
<212> PRT
<213> Artificial Sequence

<220>
<223> Description of Artificial Sequence: caveolin
      binding motif

<400> 74
Trp Xaa Trp Xaa Xaa Xaa Xaa Trp Xaa Trp
1               5                   10

<210> 75
<211> 10
<212> PRT
<213> Artificial Sequence

<220>
<223> Description of Artificial Sequence: caveolin
      binding motif

<400> 75
Trp Xaa Phe Xaa Xaa Xaa Xaa Tyr Xaa Phe
1               5                   10

<210> 76
<211> 10
<212> PRT
<213> Artificial Sequence

<220>
<223> Description of Artificial Sequence: caveolin
      binding motif

<400> 76
Trp Xaa Tyr Xaa Xaa Xaa Xaa Tyr Xaa Phe

1                 5                   10

<210> 77
<211> 10
<212> PRT
<213> Artificial Sequence

<220>
<223> Description of Artificial Sequence: caveolin
      binding motif

<400> 77
Trp Xaa Trp Xaa Xaa Xaa Xaa Tyr Xaa Phe
  1                 5                   10

<210> 78
<211> 10
<212> PRT
<213> Artificial Sequence

<220>
<223> Description of Artificial Sequence: caveolin
      binding motif

<400> 78
Trp Xaa Phe Xaa Xaa Xaa Xaa Trp Xaa Phe
  1                 5                   10

<210> 79
<211> 10
<212> PRT
<213> Artificial Sequence

<220>
<223> Description of Artificial Sequence: caveolin
      binding motif

<400> 79
Trp Xaa Tyr Xaa Xaa Xaa Xaa Trp Xaa Phe
  1                 5                   10

<210> 80
<211> 10
<212> PRT
<213> Artificial Sequence

<220>
<223> Description of Artificial Sequence: caveolin
      binding motif

<400> 80
Trp Xaa Trp Xaa Xaa Xaa Xaa Trp Xaa Phe
  1               5                   10

<210> 81
<211> 10
<212> PRT
<213> Artificial Sequence

<220>
<223> Description of Artificial Sequence: caveolin
      binding motif

<400> 81
Trp Xaa Tyr Xaa Xaa Xaa Xaa Phe Xaa Phe
  1               5                   10

<210> 82
<211> 10
<212> PRT
<213> Artificial Sequence

<220>
<223> Description of Artificial Sequence: caveolin
      binding motif

<400> 82
Trp Xaa Trp Xaa Xaa Xaa Xaa Phe Xaa Phe
  1               5                   10

<210> 83
<211> 10

<212> PRT
<213> Artificial Sequence

<220>
<223> Description of Artificial Sequence: caveolin
      binding motif

<400> 83
Trp Xaa Tyr Xaa Xaa Xaa Xaa Phe Xaa Tyr
  1               5                  10

<210> 84
<211> 10
<212> PRT
<213> Artificial Sequence

<220>
<223> Description of Artificial Sequence: caveolin
      binding motif

<400> 84
Trp Xaa Trp Xaa Xaa Xaa Xaa Phe Xaa Tyr
  1               5                  10

<210> 85
<211> 10
<212> PRT
<213> Artificial Sequence

<220>
<223> Description of Artificial Sequence: caveolin
      binding motif

<400> 85
Trp Xaa Tyr Xaa Xaa Xaa Xaa Phe Xaa Trp
  1               5                  10

<210> 86
<211> 10
<212> PRT
<213> Artificial Sequence

<220>
<223> Description of Artificial Sequence: caveolin
      binding motif

<400> 86
Trp Xaa Trp Xaa Xaa Xaa Xaa Phe Xaa Trp
  1               5                  10

## Description

### Field of the invention

The present invention is related to the use of a pharmaceutical composition for the prevention and/or the treatment of various diseases and pathologies of mammals, including the human, such as ischemic heart and peripheral vascular including cerebral diseases and tumour development and for wound healing.

The present invention is also related to a method of study, testing and/or screening of new compound or compositions which may be used for the treatment or the prevention of said various diseases and pathologies.

### Technological background of the invention

A therapeutic angiogenesis favour the development of collateral vessels to revascularise ischemic territories. Administration of angiogenic cytokines was recently proposed as an alternative to surgery or percutaneous transluminal coronary angioplasty (PTCA) for patients suffering from ischemic cardiomyopathy. This approach is hampered by two major limitations:
- the presence of a diseased and dysfunctional endothelium in ischemic tissue alters its sensitivity to angiogenic cytokines and renders their use difficult to standardise; many contradictory reports on angiogenic properties of these cytokines stemmed from inconsistencies of their effects in vitro versus in vivo, or according to the dose used;
- the need to maintain a high local concentration of cytokines is a special challenge given numerous side effects, i.e. hypotension, consecutive to the administration of high bolus doses of angiogenic factors ; alternatively, the repetitive administration of recombinant proteins is too costly.

It is also known that the decrease and even more the blocking of blood circulation in specific parts of the human body may induce the necrosis of tissues that are no longer irrigated by specific affected blood vessels. It is known that coronary vessels supplying oxygen and nutrients are essential for the irrigation of the cardiac muscle. A coronary insufficiency (for instance, induced by atherosclerosis) can be corrected by angioplasty with dilatation of the vessel obstructed by a cholesterol deposit or by surgery by bridging the non-affected portions of the vessel. However, said techniques present several drawbacks or limitations like their costs, possible reocclusion of the dilated vessel or side effects of said surgery.

NO is produced in endothelial cells by a Ca²⁺/calmodulin-dependent enzyme, the endothelial isoform nitric oxide synthase (eNOS). It has been recently shown that eNOS is located in plasmalemmal invaginations termed caveolae, and specifically interacts with cell-specific isoforms of caveolin, the structural protein of caveolae (Feron et al., 1996). Further experiments have revealed that this association leads to the inhibition of the enzyme activity and that a stable protein-protein interaction takes place between both proteins (Michel et al., 1997a; Feron et al., 1998a), i.e. eNOS and caveolin-1 in endothelial cells or eNOS and caveolin-3 in cardiac myocytes. Consensus sequences were identified within both proteins: the *scaffolding domain of caveolin,* a juxtamembrane region of 20 amino acids in the C-terminal moiety of caveolin (Michel et al., 1997b), and a putative *caveolin binding motif,* a peptide sequence rich in aromatic residues localized in the oxygenase domain of eNOS (Feron et al., 1998a; Garcia Cardena et al., 1997). In order for eNOS to be fully activated, caveolin must dissociate from the enzyme. The ubiquitous Ca²⁺ regulatory protein calmodulin (CaM) disrupts the heteromeric complex formed between eNOS and caveolin in a Ca²⁺-dependent fashion (Michel et al., 1997a; Feron et al., 1998b): caveolin serves as a competitive inhibitor of CaM-dependent eNOS activation (Michel et al., 1997b; Michel et al., 1997).

As a corollary the activity of eNOS in cells is influenced by changes in the equilibrium between these two protein-protein interactions upon stoechiometric changes in the abundance of either of these partners. It has been shown that in cardiac myocytes, the transfection of a caveolin-3 construct or the loading of a synthetic peptide corresponding to the caveolin scaffolding domain leads to the decrease in NO production at the basal level as well as following agonist stimulation (Feron et al., 1998c). Furthermore, exposure to high cholesterol concentrations decreases NO production through an upregulation of the abundance of endogenous caveolin-1 (mediated by a sterol regulatory element in its promoter region) and the subsequent inhibitory heterocomplex formation with eNOS (Feron et al. 1999). Functional consequences following changes in the abundance of caveolin-3 or the use of caveolin-like peptides have been illustrated in models of cultured myocytes *in vitro,* with alterations of the sensitivity of their beating rate to parasympathetic stimulation.

Due to its complex biochemistry in biological milieu, NO release from exogenous donor drugs does not recapitulate all the effects of NO as produced endogenously by nitric oxide synthases. Therefore, it is necessary to develop specific biochemical processes that are involved in the promotion or the inhibition of nitric oxide release, which finds applications in the prevention and/or the treatment of various diseases and pathologies like ischemic heart and peripheral vascular including cerebral diseases, wound healing and/or tumour development.

However, today, there is no published demonstration of the impact of the alterations in the abundance of caveolin-1 on the physiological behaviour of endothelial cells (downstream from their production of NO).

### State of the art

The document JP-10087698 describes the use of human heart caveolin peptide or its corresponding DNA for preventing and treating diabetes, obesity, cancer, arteriosclerosis and muscular dystrophy. This document relates to the use of the muscle-specific isoform of caveolin (or caveolin-3) but does not refer to the use of the endothelial caveolin isoform (caveolin-1).

It is also known to obtain a modulation of the NO production by the use of L-arginine-derived drugs or various drugs mixtures (as described in the documents WO98/48826, WO95/11014, US-5,767,160 and US-5,543,430) or with strategies based upon the exploitation of eNOS sequence (as described in the documents WO98/02555, JP-10136989, WO96/01902, WO93/18156), TO target atherogenesis and restenosis (as described in the document US-5,428,070).

### Aims of the invention

The present invention aims to provide new compounds, compositions and/or methods which may improve the prevention and/or the treatment of various diseases or pathologies of mammals, including the human, which do not present the drawbacks of the state of the art.

In particular, the present invention aims at the restoration of a physiological production of nitric oxide (NO) in endothelial cells, which allows a restoration of a physiological and regulated production of NO in a target dysfunctional endothelium (vs. administration of high doses of recombinant cytokines and a therapeutic action on the main effector of angiogenesis (NO) that acts downstream from many angiogenic cytokines).

A last aim of the present invention is to provide a method of study, testing and screening of new compound or compositions which may be analogs, agonists or antagonists of caveolin-1 to its active site(s) upon the endothelial isoform of nitric oxide synthase (eNOS) in order to promote or inhibit nitric oxide release, with the aim of identifying compound or composition having possible therapeutic or prophylactic properties upon various pathologies or diseases, as listed below.

### Summary of the invention

The present invention is related to the use of a compound or a pharmaceutical composition for the prevention and/or the treatment of various diseases and pathologies affecting mammals, including the human, wherein said compound or said composition allows a modulation in a patient of:
- either the endogenous caveolin-1 (present in a mammalian patient) binding to its active site(s) preferably its active site upon the endothelial isoform of nitric oxide synthase(eNOS) or other molecules such as kinases,
- or the concentration of endogenous caveolin-1.

It is meant by a modulation of the caveolin-1 binding the possibility to enhance or decrease the binding of caveolin-1 upon its active site(s), preferably upon the endothelial isoform of nitric oxide synthase (eNOS) which may advantageously result in an increase or decrease of a nitric oxide (NO) synthesis.

Preferred examples of said modulation is described hereafter.

The decrease or the increase of nitric oxide results in a modification of neo-angiogenesis which has prophylactic or therapeutic properties in specific pathologies and diseases of mammals (including the human), and preferably selected from the group consisting of: high blood pressure, cardiac insufficiency, cardiac decompensation, ischemic cardiomyopathy, dilated or post-transplantation cardiomyopathies, angina pectoris (including instable angina), coronary spasm, post-transplantation coronaropathy, hypercholesterolemia, hyperlipidemia, hypertriglyceridemia, vascular side effects of diabetes melitus (insulino-dependent or not), vascular side effects of chronic renal insufficiency (uremia), endothelial dysfunction of various origins (atherosclerosis, smoke-addiction, syndrome X, obesity, hypertension, dyslipidemia, resistance to insulin), systemic or auto-immune vasculitis, hyperhomocysteinemia, buerger angeitis, post-angioplasty coronary restenosis, peripheral vascular disease, thrombo-embolic disease, deep or superficial vein thrombosis, atherosclerosis with arterial insufficiency in a vascular area (ischemia, including cerebral ischemia, coronary ischemia, ...), pulmonary arterial hypertension, side effects of hemodialysis or peritoneal dialysis, various neoplastic diseases (including carcinogenesis, tumoral development and metastases proliferation, resistance of malignant neoplastic tumors to radio or chemotherapy, angiosarcoma, proliferative retinopathies, wound healing or a mixing thereof.

Another aspect of the present invention is related to a method of study, testing and/or screening of a (known or unknown) compound or (known or unknown) compositions which could be used in the treatment or the prevention of the above-identified diseases or pathologies, said methods comprising the step of putting said compounds or compositions into contact with the active site(s) of interaction of caveolin-1 preferably with the endothelial isoform of nitric oxide synthase (eNOS), possibly in a competition test with caveolin-1 to its active site(s) and the step of identifying (measuring) if said compound or compositions promote(s) or inhibit(s) nitric oxide release and could be used as agonist(s) or antagonist(s) of caveolin-1 to its active sites, preferably upon the endothelial isoform of nitric oxide synthase.

The last aspect of the present invention concerns unknown compound or compositions identified and recovered by said study, testing and/or screening method and which are preferably used to promote or inhibit nitric oxide release preferably by nitric oxide synthase (eNOS) and may find possible therapeutic and/or prophylactic properties for the above-identified pathologies and diseases or which are used in the manufacture of a medicament in the prevention and/or the treatment of the above-identified pathologies and/or diseases.

The composition according to the invention may comprise one or more active compounds having possible synergetic effects and a suitable pharmaceutical carrier or adjuvant that may vary according to the mode of administration.

Said suitable pharmaceutical carrier or adjuvant is a common pharmaceutical carrier or adjuvant well known by the person skilled in the art and used to increase or modulate the therapeutical and/or prophylactic effects of the active compounds according to the invention and/or to decrease the possible side effects of said compound(s).

The pharmaceutical composition according to the invention is prepared according to the methods generally applied by pharmacists and may include solid or liquid non-toxic and pharmaceutically acceptable carrier(s) or vehicle(s).

The percentage of active product / pharmaceutically suitable carrier can vary within very large ranges, only limited by the tolerance and the level of the habit forming effects of the composition to the mammal (including the human). These limits are particularly determined by the frequency of administration.

### Detailed description of the invention

Exposure to high cholesterol concentrations decreases NO production through an upregulation of the abundance of endogenous caveolin-1. By reducing the cholesterol content in endothelial cells, the inventors have discovered unexpectedly that in endothelial cells, the caveolin-1 down-regulation is correlated with an increase in NO release, probably by a stoechiometric regulation of eNOS activity by a cellular pool of caveolin.

Therefore, the inventors have discovered for the first time that it is possible, by a modulation of caveolin-1 concentration in a patient or a modulation of the interaction(s) between endogenous caveolin-1 and its active site(s) preferably upon the endothelial isoform of nitric oxide synthase (eNOS), or other molecules such as kinases to promote or inhibit advantageously nitric oxide release which may find prophylactic or therapeutical application in various pathologies and/or diseases.

Said modulation is obtained by known or unknown compounds or compositions that are or comprise analogs, antagonists or agonists of caveolin-1 to its active site(s) upon various molecules, preferably upon the endothelial isoform of nitric oxide synthase.

As caveolin-1 is a peptide (see enclosed SEQ ID NO 1), said analogs, agonists or antagonists can be derivatives of said peptide, peptidomimetics or homologous peptides that comprise the deletion or the replacement of one or more amino acids in the original caveolin-1 sequence, or antibodies directed against the ligand binding site epitopes of the active site(s) upon the endothelial isoform of nitric oxide synthase, or anti-idiotypic antibodies directed against particular antibodies directed against the specific portions of caveolin-1 (scaffolding domain A and/or B of caveolin-1) binding the active site(s) of caveolin-1 upon the endothelial isoform of nitric oxide synthase. Said antibodies can be raised to caveolin-1 fragments and analogs both in the unnatural oCcuring form or in the unrecombined form.

Examples of said analog or agonist is the scaffolding domains of caveolin-1 or portions thereof.

Said antagonists or agonists can be also made of genetic sequences (possibly comprised in a recombinant expression vector), either an antisense nucleotide sequence (like the antisense sequence (SEQ ID NO 5)) that is able to hybridise with a corresponding sequence encoding the partial or total amino acid sequence of caveolin-1, preferably comprised into a vector, under the control of a regulatory sequence that allows its expression (preferably its high expression) in a transfected cell (preferably in an endothelial transfected cell in high amounts).

The vectors that can be used for the integration of said genetic sequence are plasmids or viral vectors (such as adenoviruses), which are able to be used for the transfection of endothelial cells *in vitro, in vivo* or *ex-vivo.*

Other examples of agonists or antagonists according to the invention are short peptidic sequences corresponding to the active sites (having preferably at least the common pattern SEQ ID NO 4, more preferably the pattern SEQ ID NO 6 to 86 upon eNOS to the caveolin-1 scaffolding domains A and B (described in the following sequences SEQ ID NO 2 and SEQ ID NO 3) or are peptidic sequences comprising the partial or total sequence of said caveolin-1 scaffolding domains A or B. Thse agonists or antagonists include the corresponding nucleotidic sequences encoding said peptidic sequences of caveolin-1 or eNOS preferably comprised into a vector as described above.

It is also possible to obtain said modulation of interactions between endogenous caveolin-1 and its active site(s), preferably upon the endothelial isoform of nitric oxide synthase by drugs that affect levels of expression (and therefore abundance) or intracellular localisation of endogenous caveolin-1, including drugs such as HMGCoA reductase inhibitors (like the compound Atorvastatin® obtained from PARKE Inc.) that affect intracellular cholesterol content.

### REFERENCES

Feron O. et al., *Journal of Clinical Investigation* **103**, pp. 897-905 (1999).
Feron O. et al., *Journal of Biological Chemistry* **273**, pp. 30249-30254 (1998c).
Feron O. et al., *Journal of Biological Chemistry* **273**, pp. 3125-3128 (1998b).
Feron O. et al., *Biochemistry* **37***,* pp. 193-200 (1998a). Michel T. & Feron O., *Journal of Clinical Investigation* **100**, pp. 2310-2316 (1997).
Michel JB. et al., *Journal of Biological Chemistry* **272**, pp. 25907-25912 (1997b).
Michel JB. et al., *Journal of Biological Chemistry* **272**, pp. 15583-15586 (1997a).
Feron O. et al., *Journal of Biological Chemistry* **271**, pp. 22810-22814 (1996).
Garcia Cardena et al., *Journal of Biological Chemistry* **272**, PP. 25437-25440 (1997).

## Claims

1. Use of a compound or a pharmaceutical composition for the prevention and/or the treatment of ischemic heart and peripheral vascular diseases including cerebral diseases, tumour development and wound healing, wherein said compound or composition being able to modulate positively or negatively in a patient:
- either the endogenous caveolin-1 binding to its active site(s),
- or the concentration of endogenous caveolin-1.

2. Use according to claim 1, wherein the compound or composition being able to modulate positively or negatively in a patient:
- the endogenous caveolin-1 binding to its active site(s) upon the endothelial isoform nitric oxide synthase (eNOS).

3. Use according to the claim 1 or 2, wherein said compound or said pharmaceutical composition consists or comprises an analog of caveolin-1, an antagonist of caveolin-1 or an agonist of caveolin-1 to its binding site(s).

4. Use according to claim 3, wherein said antagonist is an antisense nucleotide sequence able to hybridise with a corresponding nucleotide sequence encoding the caveolin-1.

5. Use according to the claim 4, wherein the antagonist is the antisense sequence SEQ ID NO 5.

6. Use according to claim 3, wherein the antagonist is a molecule comprising at least the amino acid sequence pattern of SEQ ID NO 4, preferably the amino acid sequence pattern of SEQ ID NO 6to SEQ ID NO 86.

7. Use according to claim 3, wherein said agonist is a nucleotide sequence encoding the partial or total amino acid sequence of caveolin-1 integrated into a vector allowing its expression, preferably its high expression, in a cell *in vitro, in vivo* or *ex vivo.*

8. Use according to claim 3, wherein the agonist is a compound comprising or encoding the caveolin-1 scaffolding domains A and/or B or portions thereof able to bind the active site(s) of caveolin-1, preferably upon the endothelial isoform of nitric oxide synthase, said compound being possibly incorporated in a suitable vector.

9. Use according to claim 1, wherein the compound or the composition being able to modulate negatively the concentration of endogenous caveolin-1 is a cholesterol reducing drugs, such as HMGCoA reductase inhibitor.

10. Method of study, testing and/or screening of a compound or a composition which can be used for the prevention and/or the treatment of ischemic heart and peripheral vascular including cerebral diseases, tumour development and wound healing, comprising the step of putting said known or unknown compound or composition into contact with the active site(s) of caveolin-1, preferably upon the endothelial isoform of nitric oxide synthase (eNOS), possibly in a competition with caveolin-1 to said active site(s), and to measure if said compound or composition promotes or inhibits nitric oxide release and possibly comprising the step of recovering said unknown compound or composition.

11. Unknown compound or composition identified and recovered by the study, testing and/or screening method of claim 10 and which may be used as an agonist or an antagonist of caveolin-1 to its active site(s) upon the endothelial isoform of nitric oxide synthase (eNOS).
